# EUROPEAN PATENT APPLICATION

(11) **EP 4 822 014 A1**
(43) Date of publication of application: **14.10.2026**
(21) Application number: 24900585.1
(22) Date of filing: 02.12.2024
(51) Int. Cl.: C12N 5/071, C12N 5/077, C12N 5/0775

(54) **METHOD FOR PRODUCING AGGREGATES OF CELLS, POPULATION OF AGGREGATES, AND METHOD FOR CULTURING CELLS**

(30) Priority: 04.12.2023 JP 2023204841
(71) Applicant: Cuorips Inc., Tokyo 103-0023 (JP)
(72) Inventor: SAWA Yoshiki, Tokyo 103-0023 (JP); LIU Li, Suita-shi, Osaka 565-0871 (JP); LI Junjun, Suita-shi, Osaka 565-0871 (JP); ITO Emiko, Tokyo 103-0023 (JP); TAKEDA Maki, Tokyo 103-0023 (JP)
(74) Representative: Grünecker Patent- und Rechtsanwälte PartG mbB
(86) International application number: PCT/JP2024/042576
(87) International publication number: WO 2025/121291

(57) **Abstract**

In one embodiment, cells 12 are suspended within a hollow capsule 11 composed of a semipermeable membrane, and the hollow capsule 11 is incubated in a liquid culture medium to induce aggregation of the cells 12 within the hollow capsule 11. The cells 12 comprise a mixture of dispersed cells of interest 14 and auxiliary cells 15 that assist aggregation of the cells of interest 14. Cells of interest 14 aggregate more readily when auxiliary cells 15 mediate than when they are absent. In another embodiment, dispersed cells 12, comprising at least stromal cells, are suspended within a hollow capsule 11 composed of a semipermeable membrane, and the hollow capsule 11 is incubated in a liquid culture medium to induce aggregation of the cells within the hollow capsule 11. Thereby, a cell aggregate 21 is produced.

## Description

### TECHNICAL FIELD

The present invention relates to cell aggregation.

### BACKGROUND ART

Figure 1a of Patent Document 1 and Figure 1a of Patent Document 2 disclose culturing cardiomyocytes in an isotonic aqueous solution encapsulated by an outer hydrogel layer. Non-Patent Documents 1 to 3 disclose culturing pluripotent stem cells within hollow capsules. Patent Document 3 discloses culturing mixtures of various cell types and undifferentiated cells on a gel-like support. The entire contents of Patent Documents 1 and 2 and Non-Patent Documents 1 to 3 are incorporated herein by reference.

### PRIOR ART

### [Patent Documents]

[Patent Document 1] International Publication No. WO2022/058615
[Patent Document 2] International Publication No. WO2022/058617
[Patent Document 3] International Publication No. WO2015/129822

### [Non-Patent Literature]

[Non-Patent Document 1] NEWS LETTER, Division of Biotechnology, The Chemical Society of Japan, Vol. 24, No.1 (2020.08.01), pp.17 to 20, [retrieved on 2023-06-26]. Retrieved from <https://bio.chemistry.or.jp/_userdata/NL24_1.pdf>
[Non-Patent Document 2] Horiguchi, I., Chowdhury, M.M., Sakai, Y. and Tabata, Y. (2014), Proliferation, morphology, and pluripotency of mouse induced pluripotent stem cells in three different types of alginate beads for mass production. Biotechnol Progress, 30: 896-904. [retrieved on 2023-06-26]. Retrieved from <https://doi.org/10.1002/btpr.1891>.
[Non-Patent Document 3] Horiguchi, I., Sakai, Y. Alginate Encapsulation of Pluripotent Stem Cells Using a Co-axial Nozzle. J. Vis. Exp. (101), e52835, (2015) [retrieved on 2023-06-26]. Retrieved from <https://doi:10.3791/52835>.

### SUMMARY OF THE INVENTION

### [Problem to be Solved by Invention]

The present invention provides a means for efficiently inducing aggregation of specific cells.

### [Means for Solving the Problem]

<1> A method for producing a cell aggregate, comprising:
   suspending cells within a hollow capsule composed of a semipermeable membrane; and incubating the hollow capsule in a liquid culture medium so as to aggregate the cells within the hollow capsule, wherein the cells comprise a mixture of dispersed cells of interest and auxiliary cells that assist aggregation of the cells of interest, and wherein the cells of interest aggregate more readily in the mediation of the auxiliary cells than in their absence.
<2> The above method for producing a cell aggregate, wherein
   the cells of interest are anyone of cardiomyocytes, hepatocytes, and other parenchymal cells, and/or wherein the auxiliary cells are at least one of mesenchymal stem cells, fibroblasts, vascular endothelial cells, and other stromal cells.
<3> A method for producing a cell aggregate, comprising:
   suspending dispersed cells within a hollow capsule composed of a semipermeable membrane; and
   incubating the hollow capsule in a liquid culture medium so as to aggregate the cells within the hollow capsule, wherein
   the cells comprise at least stromal cells.
<4> The above method for producing a cell aggregate, wherein the stromal cells are at least one of mesenchymal stem cells, fibroblasts, vascular endothelial cells, and other stromal cells.
<5> The above method for producing a cell aggregate, wherein the cells further include anyone of cardiomyocytes, hepatocytes, and other parenchymal cells.
<6> A method for producing a cell aggregate, comprising:
   enclosing a cell-containing gel, which is made of a material different from the semipermeable membrane, within the semipermeable membrane;
   dissolving the gel; and
   allowing the dissolved gel to elute outward through the semipermeable membrane to form the hollow capsule, whereby the cells are retained within the hollow capsule.
<7> The above method for producing a cell aggregate, wherein
   the gel is made of alginate containing divalent cations, and the gel is dissolved by treatment with a chelating agent.
<8> The above method for producing a cell aggregate, wherein
   the cell-containing gel is formed into beads by extruding the gel while enveloping it with a gas stream, and the resulting beads are then encapsulated with the semipermeable membrane.
<9> The above method for producing a cell aggregate, comprising:
   rupturing the hollow capsule by passing it through a tube having an inner diameter smaller than the outer diameter of the hollow capsule, and separating and recovering the aggregated cells from the ruptured hollow capsule.
<10> The above method for producing a cell aggregate, wherein
   the semipermeable membrane comprises poly-L-lysine.
<11> The above method for producing cell aggregates, wherein
   the hollow capsule is incubated under rotary agitation.
<12> The above method for producing a cell aggregate, further comprising incubating the hollow capsules to allow proliferation of the aggregated cells.
<13> A population of aggregates, wherein:
   the mean diameter of aggregates in the population is 70 to 150 µm; and
   the standard deviation of aggregate diameters in the population is 7 to 20 µm; wherein
   each aggregate is either:
      (a) formed by cells of interest aggregated with auxiliary cells, the mediation of the auxiliary cells promoting aggregation of the cells of interest compared to when the auxiliary cells are absent; or
      (b) formed by aggregation of stromal cells.
<14> The above population of aggregates, wherein
   each aggregate is individually enclosed in a hollow capsule composed of a semipermeable membrane, and
   the aggregate is suspended in the liquid contained within the hollow capsule.
<15> A cell culture method, comprising:
   suspending cells within a hollow capsule composed of a semipermeable membrane; and
   incubating the hollow capsules in a liquid culture medium until the cells aggregate at least within the hollow capsules, wherein the cells comprise a mixture of dispersed cells of interest and auxiliary cells that assist aggregation of the cells of interest, and wherein the cells of interest aggregate more readily in the mediation of the auxiliary cells than in their absence.
<16> A cell culture method, comprising:
   suspending dispersed cells within a hollow capsule composed of a semipermeable membrane; and
   incubating the hollow capsules in a liquid culture medium until the cells aggregate within the hollow capsules, wherein
   the cells comprise at least stromal cells.

### [Effect of the Invention]

The present invention provides a means for efficiently aggregating specific cells.

### BRIEF DESCRIPTION OF DRAWINGS

[Figure 1] Schematic illustration of cell aggregation within a capsule
[Figure 2] Schematic illustration of cell aggregation within a capsule
[Figure 3] Schematic illustration of bead formation
[Figure 4] Schematic illustration of capsule formation
[Figure 5] Schematic illustration of aggregate recovery
[Figure 6] Schematic illustration of a population of aggregates
[Figure 7] Microscopic observation photograph of a hollow capsule
[Figure 8] Microscopic observation photograph of formed organoids
[Figure 9] Histogram of organoid sizes
[Figure 10] Histogram of capsule and organoid sizes
[Figure 11] Histogram of capsule and organoid sizes
[Figure 12] Microscopic observation photograph of released organoids
[Figure 13] Microscopic observation photograph of fluorescently stained organoids
[Figure 14] Microscopic observation photograph of cardiomyocyte aggregation
[Figure 15] Microscopic observation photograph of aggregation between cardiomyocytes and mesenchymal stem cells
[Figure 16A] Microscopic observation photograph of myocardial markers on myocardial spheroids
[Figure 16B] Microscopic observation photograph of myocardial markers on myocardial organoids
[Figure 17] Graph of fluorescence intensity for each marker in Figures 16A and 16B
[Figure 18] Histogram of CD90 marker expression intensity
[Figure 19] Graph of TGF-β1 and VEGF secretion levels
[Figure 20] Analysis results of contractile force of myocardial spheroids
[Figure 21] Analysis results of contractile force of myocardial organoids
[Figure 22] Analysis results of amplitude of contractile force and amplitude of contraction velocity
[Figure 23] Analysis results of cell survival under hypoxic conditions
[Figure 24] Microscopic observation photograph of myocardial organoids on day 2 of culture
[Figure 25] Microscopic observation photograph of myocardial organoids on day 8 of culture
[Figure 26] Results of the count of organoids contained in wells
[Figure 27] Microscopic observation photograph of spheroids derived from adipose tissue-derived mesenchymal stem cells
[Figure 28] Microscopic observation photograph of liver organoids

### DETAILED DESCRIPTION OF EMBODIMENTS

### <Aggregation of cells of interest that are difficult to aggregate>

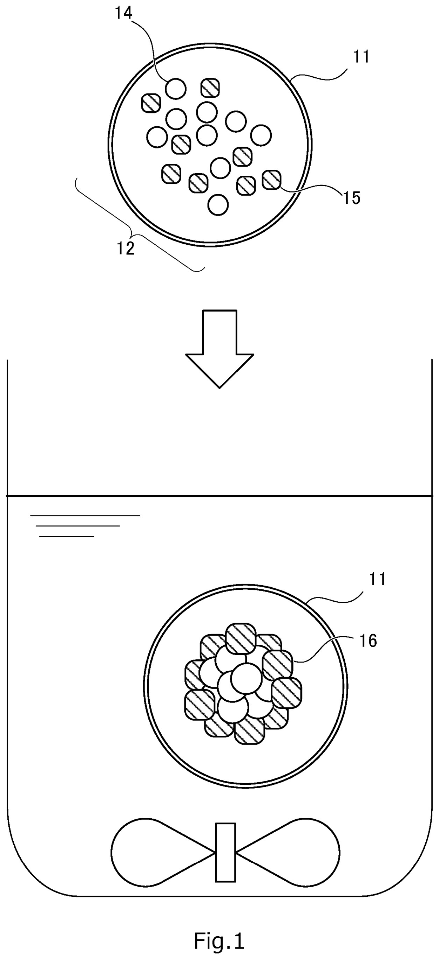

Figure 1 schematically illustrates one embodiment of cell aggregation within a capsule. As shown in the upper part, cells 12 are suspended within a hollow capsule 11 formed of a semipermeable membrane. As shown in the lower part, incubating the hollow capsule 11 in liquid culture medium causes the cells 12 to aggregate inside the hollow capsule 11. As a result, an aggregate 16 is obtained within the hollow capsule 11.

In Figure 1, the aggregate 16 may be composed of multiple cell types and may constitute a three-dimensional tissue-like structure having organ-like functions. Such a tissue-like structure is referred to in this embodiment as an organoid. In another aspect, a three-dimensional tissue-like structure composed of a single cell type is referred to in this embodiment as a sphere/spheroid.

As shown in the upper part of Figure 1, the cells 12 are a mixture of cells of interest 14, represented by circles, and auxiliary cells 15, represented by rounded squares. Cells of interest 14 are dispersed prior to incubation and are the cells intended to aggregate after incubation. Cells of interest 14 are relatively difficult to aggregate. Auxiliary cells 15 are cells that assist the aggregation of cells of interest 14 during incubation. In this embodiment, cells of interest 14 aggregate more readily when auxiliary cells 15 mediate than when they are absent.

In the embodiment shown in Figure 1, the cells of interest 14 may be cardiomyocytes, hepatocytes, or other parenchymal cells. The auxiliary cells 15 may be mesenchymal stem cells, fibroblasts, vascular endothelial cells, or other stromal cells.

As shown in Figure 1, the aggregate 16 is enclosed by the hollow capsule 11 composed of a semipermeable membrane. The aggregate 16 consists of cells of interest 14 and auxiliary cells 15. The aggregate 16 is suspended in the liquid culture medium or buffer solution that fills the hollow capsule 11. As described later, the aggregate 16 may be released from the hollow capsule 11.

In one embodiment illustrated in Figure 1, the hollow capsule 11 is incubated but the aggregated cells are not allowed to proliferate. In other words, proliferation and growth of the cells within the aggregate 16 are not the objective of the incubation. Rather, incubation is performed only for a short time with the sole purpose of inducing cell aggregation. This reduces variation in the size of aggregate 16. Moreover, it prevents substantial changes in the ratio of cells of interest 14 to auxiliary cells 15 within aggregate 16. During incubation, the hollow capsule 11 may be rotated and stirred. Because the aggregate 16 is protected within the hollow capsule 11, it is less susceptible to physical forces produced by rotation and stirring, such as shear stress.

### <Aggregation of stromal cells>

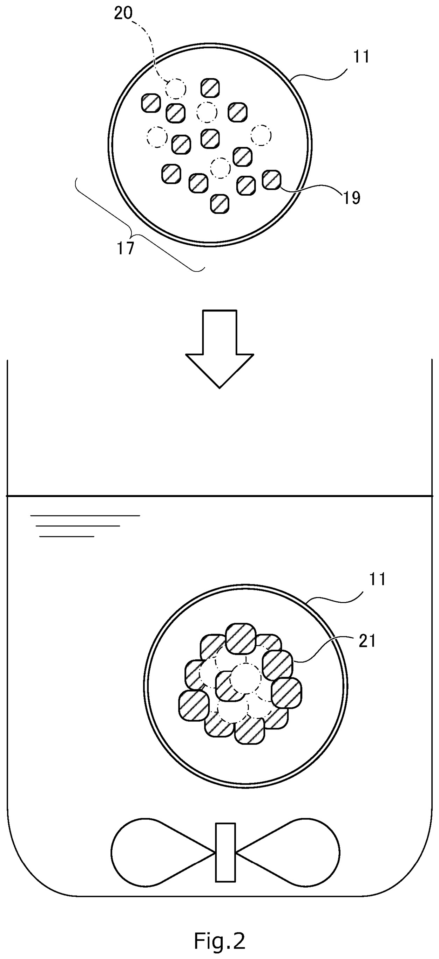

Figure 2 schematically illustrates another embodiment of cell aggregation within a capsule. As shown in the upper part, cells 17 dispersed within the hollow capsule 11 formed of a semipermeable membrane are suspended. As shown in the lower part, incubating the hollow capsule 11 in liquid culture medium causes the cells 17 to aggregate inside the hollow capsule 11. At this time, the hollow capsule 11 may be rotated and stirred. Thus, an aggregate 21 is obtained within the hollow capsule 11. The aggregate may be a three-dimensional tissue structure having organ-like functions, *i.e.*, an organoid.

As shown in the upper part of Figure 2, the cells 17 comprise at least stromal cells 19, represented by rounded squares. The stromal cells 19 may be mesenchymal stem cells, fibroblasts, vascular endothelial cells, or other stromal cells. The cells 17 may further include parenchymal cells 20. The parenchymal cells 20 may be cardiomyocytes, hepatocytes, or other parenchymal cells. The cells 17 may also consist solely of stromal cells.

In Figure 2, incubation of the cells 17 placed within the hollow capsule 11 can be carried out by the method described with reference to Figure 1. The aggregate 21 is enclosed within the hollow capsule 11 composed of a semipermeable membrane. The aggregate 21 comprises at least stromal cells 19. The aggregate 21 is suspended in the liquid culture medium contained within the hollow capsule 11. As described later, the aggregate may be released from the hollow capsule 11. The aggregate 21 may be an organoid or a spheroid.

### <Placing cells into a hollow capsule>

Next, an example of a capsule formation process used in any of the above aggregation modes is described. Here, cell 12, consisting of cells of interest 14 and auxiliary cells 15 as shown in Figure 1, is used as an example. The following method can also be applied to cell 17 composed of stromal cells as shown in Figure 2.

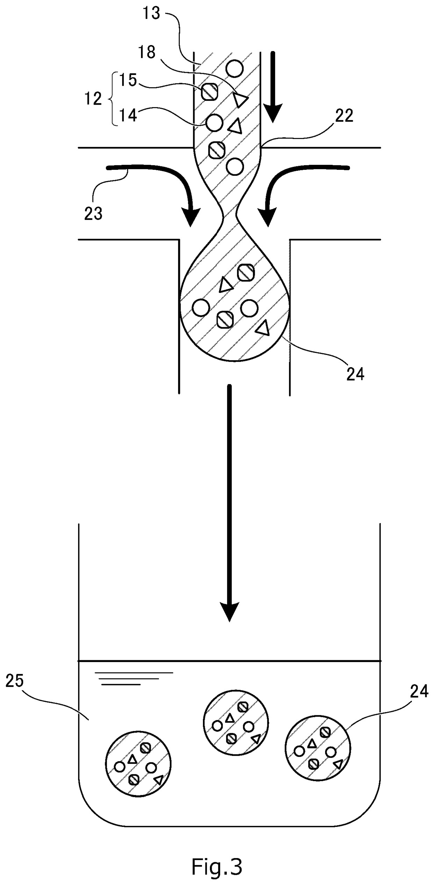

Figure 3 schematically illustrates one embodiment of a bead-formation step used when encapsulating cells. In this process, gel 13 containing cells 12 is continuously extruded through nozzle 22 while being enveloped by gas flow 23, thereby forming beads 24. Increasing the number of nozzles 22 enables bead production at higher throughput.

In Figure 3, the cross-sectional diameter of nozzle 22 is preferably from 100 µm to 1000 µm, and may be, for example, 200, 300, 400, 500, 600, 700, 800, or 900 µm. Unless otherwise specified, the term "diameter" in this embodiment refers to the distance across.

In Figure 3, the gas in gas flow 23 is an inert gas, for example nitrogen. The gas pressure need only be higher than atmospheric pressure (101.325 kPa under standard conditions). The difference between the gas pressure and atmospheric pressure is preferably from 0 kPa to 100 kPa, and may be, for example, 10, 15, 20, 25, 30, 35, 40, 45, 50, 55, 60, 65, 70, 75, 80, 85, or 90 kPa.

As shown in Figure 3, cells of interest 14 and auxiliary cells 15 are dispersed within gel 13. The gel further contains nutrients 18 dissolved therein that are necessary for culturing these cells. The gel matrix is preferably a material with low cytotoxicity, such as alginate. When gel 13 is an alginate/water system, beads 24 composed of gel 13 can be produced by dropping a mixture of sodium alginate and the cell suspension into liquid 25. The sodium alginate concentration may be from 0.5 to 5.0 weight percent, and may be, for example, 0.75, 1.0, 1.1, 1.2, 1.3, 1.4, 1.5, 1.6, 1.7, 1.8, 1.9, 2.0, or 3.0 weight percent.

In Figure 3, the diameter of beads 24 can be adjusted based on, for example, the cross-sectional diameter of nozzle 22, the pressure of gas flow 23, or the concentration of gel 13.

In Figure 3, the density of cells 12 per unit volume of beads 24 may be from 0.1x10⁶ cells/mL to 10x10⁶ cells/mL, and may be, for example, 0.2, 0.5, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, or 4.0x10⁶ cells/mL.

As shown in the lower part of Figure 3, beads 24 are kept in a liquid 25 suitable for maintaining their shape. For example, when gel 13 is alginate, an ionic crosslinking agent such as a divalent cation (e.g., Ca²⁺) is required to maintain bead shape. Liquid 25 may be an aqueous solution of a divalent cation salt, for example CaCl₂. In liquid 25, sodium in the alginate is exchanged for the divalent cation, yielding beads 24 composed of crosslinked alginate.

As shown in Figure 3, using gas flow 23 enables the production of numerous beads 24 with a uniform size distribution. Consequently, hollow capsules with the uniform size distribution described below can be produced rapidly and in large quantities.

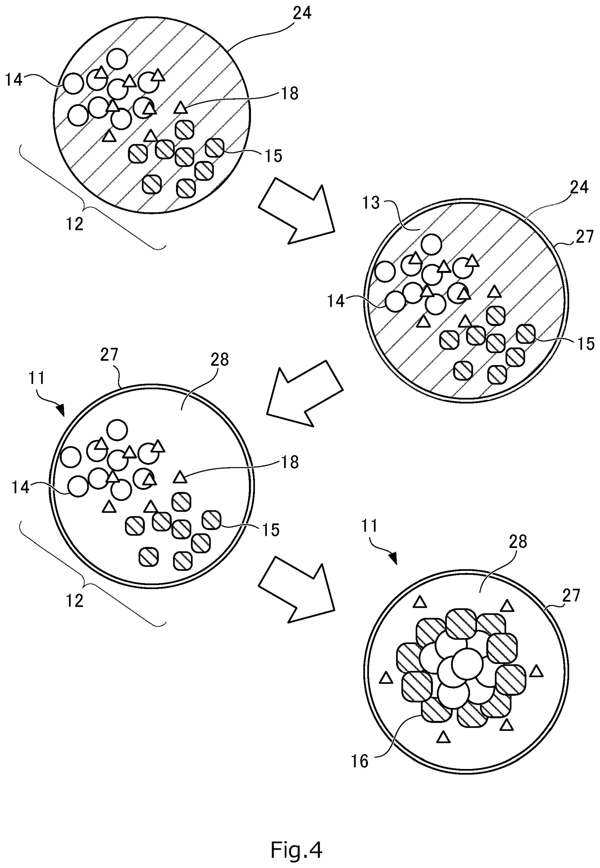

Figure 4 schematically illustrates the process for obtaining hollow capsules from the beads. First, as shown in the upper part of the figure, the beaded gel 13, *i.e.*, the beads 24, is wrapped with a semipermeable membrane 27. In one embodiment, the semipermeable membrane 27 is made of a material different from the gel 13. Viewed from the gel 13 side, the gel 13 is made of a material different from the semipermeable membrane 27.The semipermeable membrane 27 may be poly-L-lysine (PLL). For example, the surface of the beads 24 may be coated with a semipermeable membrane 27 composed of poly-L-lysine by incubating the beads 24 in a poly-L-lysine aqueous solution.

The concentration of poly-L-lysine may be from 0.005 wt% to 5 wt%, and may be any of 0.01, 0.02, 0.05, 0.1, 0.2, 0.3, 0.4, 0.5, 0.6, 0.7, 0.8, 0.9, 1.0, and 2.0 wt%.

The incubation atmosphere may be under conditions suitable for cell culture, for example, at 37°C under a 5% CO₂ atmosphere.

Next, as shown in the middle panel of Figure 4, the gel 13 is dissolved. The beads 24 disappear, leaving the cells 12 behind. The dissolved components of the gel 13 are eluted outward through the semipermeable membrane 27. This yields a hollow capsule 11 composed of the semipermeable membrane 27. The cells 12 remain within the space 28 inside the hollow capsule 11. Furthermore, the gel 13 is replaced with liquid culture medium or buffer solution.

In Figure 4, when gel 13 comprises alginate containing divalent cations, it is preferable to dissolve the gel by acting a chelating agent on gel 13. At this time, the chelating agent removes the divalent cations from gel 13.Gel 13, having lost the divalent cations, dissolves into the buffer solution on both sides of semipermeable membrane 27. The type of chelating agent is not limited; for example, sodium EDTA or sodium citrate may be used.

Next, as shown in the lower part of Figure 4, aggregates 16 are obtained within the hollow capsule 11 by incubating and aggregating the cells 12. This process is as described using Figures 1 and 2. Within the hollow capsule 11, organoids with a uniform size distribution can be formed rapidly.

In Figure 4, setting the target diameter of the hollow capsule 11 to 100, 200, 300, or 400 µm or greater facilitates the creation of uniform capsules. Alternatively, the target diameter of the hollow capsule 11 may be set to 1000, 900, or 800 µm or less. The target diameter of the hollow capsule 11 may also be any of 500, 600, or 700 µm. The target diameter of the hollow capsule 11 may also be the major axis length of the hollow capsule measured under microscopic observation. The size of the hollow capsule 11 can be adjusted based on the size of the beads 24.

In Figure 4, the target diameter of the aggregate 16 may be 10 to 1000 µm, or may be any of 20, 50, 60, 70, 80, 90, 100, 110, 120, 130, 140, 150, 160, 170, 180, 190, 200, or 500 µm. The target diameter of the aggregate 16 can be controlled by the density of cells 12 within beads 24.

### <Release from Hollow Capsules>

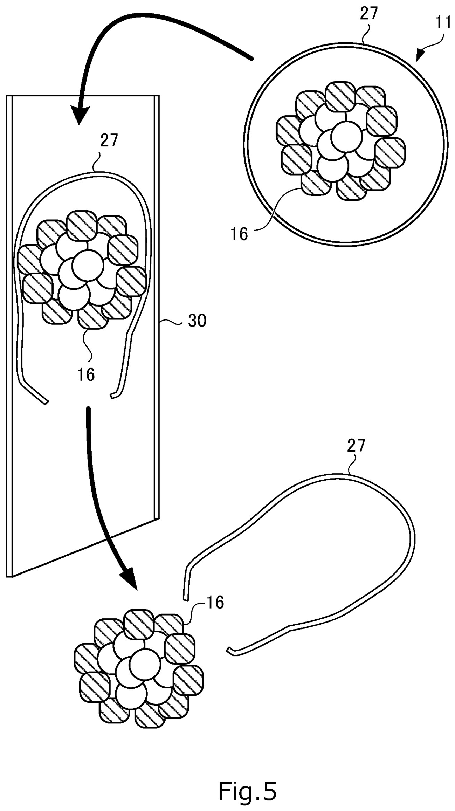

Figure 5 schematically illustrates the recovery of the aggregate 16. As shown in Figure 5, the aggregate 16 is released from the hollow capsule 11. The hollow capsule 11 is ruptured by passing it through tube 30. The shape of tube 30 is not particularly limited; for example, tube 30 may be an injection needle. Tube 30 preferably has an inner diameter smaller than the outer diameter of the hollow capsule 11. The hollow capsule 11 preferably passes through tube 30 only once. For example, the capsule may be aspirated into a syringe (not shown) via a tube thicker than tube 30, the tube connected to the syringe may then be replaced with tube 30, and the capsule may be expelled from the syringe through tube 30.

To separate and recover the aggregate 16 shown in Figure 5 from the ruptured semipermeable membrane 27, the materials may be subjected to filtration. Using a filter having an appropriate mesh size allows the aggregate 16 to be collected in the filtrate while trapping the ruptured semipermeable membrane 27 on the filter. The above method is applicable to both the aggregate 16 shown in Figure 1 and the aggregate 21 shown in Figure 2.

### <Population of Aggregates>

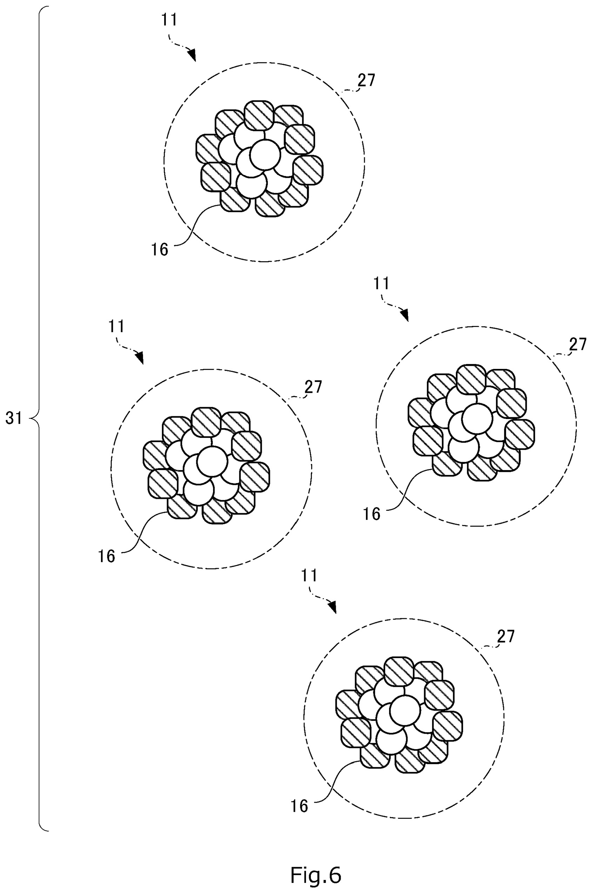

Figure 6 is a schematic diagram of a population 31 composed of aggregates 16. The aggregates 16 are produced within the hollow capsule 11. The average diameter of the aggregates 16 may be 10 to 1000 µm, or may be any of 20, 50, 60, 70, 80, 90, 100, 110, 120, 130, 140, 150, 160, 170, 180, 190, 200, or 500 µm. The standard deviation of the diameter of the aggregates 16 may be 1 to 50 µm, or may be any of 2, 3, 4, 5, 6, 7, 8, 9, 11, 12, 13, 14, 15, 16, 17, 18, 19, 20, 30, or 40 µm.

In one embodiment shown in Figure 6, the aggregate 16 comprises cells of interest that have aggregated by the mediation of auxiliary cells, as described with reference to Figure 1. In this case, cells of interest aggregate more readily when auxiliary cells are interposed than when auxiliary cells are absent. In other embodiments, the aggregate 16 comprises at least stromal cells that have aggregated, as described with reference to Figure 2.

In one embodiment shown in Figure 6, each aggregate 16 is individually enclosed within a hollow capsule 11 formed of a semipermeable membrane 27. The aggregate 16 is suspended in the liquid contained within the hollow capsule 11. In other embodiments, the aggregate 16 may not be enclosed within a hollow capsule 11 formed of a semipermeable membrane 27 and may be exposed.

### [Examples]

### <Cell Conditions>

Unless otherwise specified, cardiomyocytes were selected as the cells of interest (parenchymal cells) to be aggregated. Cardiomyocytes may be abbreviated as CM. The cardiomyocytes were not thawed from frozen stock but were harvested from cells in culture. Unless otherwise specified, cardiomyocytes were also selected as the auxiliary (stromal) cells for aggregation. Mesenchymal stem cells may be abbreviated as MSC. The mesenchymal stem cells were thawed from frozen samples. Both the cardiomyocytes and the mesenchymal stem cells were differentiated from iPS cells. When cardiomyocytes and mesenchymal stem cells were mixed, the mixing ratio was 10:1.

### <Hollow Capsule Manufacturing Conditions>

The beads were prepared as described with reference to Figure 3. Sodium alginate at a final concentration of 1.5% (w/w) was used as the gel matrix. Nitrogen gas at a pressure of 25 kPa to 75 kPa was used. The inner diameter of the nozzle extruding the gel was set to 160 µm. The hollow capsules were prepared as described with reference to Figure 4. Poly-L-lysine was used as the semipermeable membrane.

### <Incubation Conditions>

Unless otherwise specified, incubation was performed at 37°C in a 5% CO₂ atmosphere with rotary agitation, under 5% serum conditions.

### <Controlling the Gas Pressure of the Gas Flow>

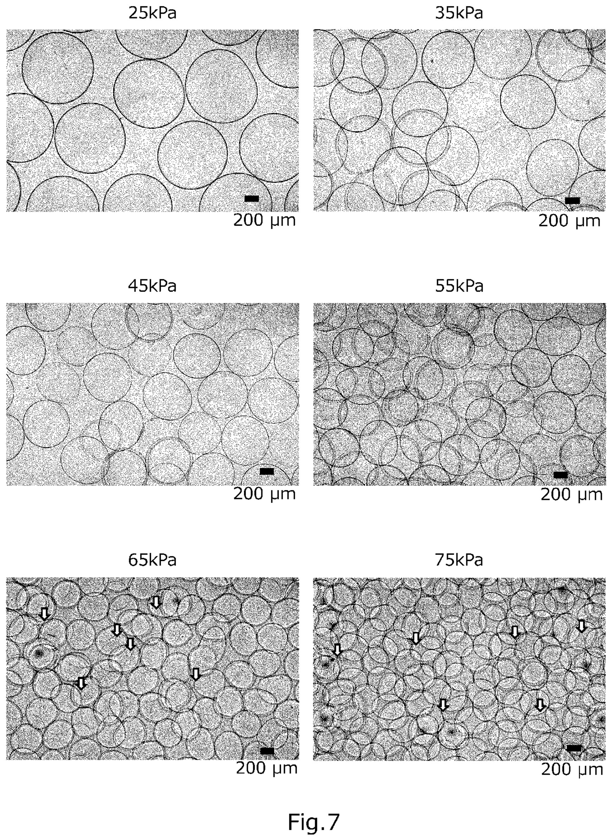

Figure 7 shows microscopic images of hollow capsules prepared from beads produced using gas streams at various pressures. The black bar at the bottom right of each image indicates a 200 µm scale. In this test, cells were not included in the beads or hollow capsules. The size of the beads and hollow capsules can be adjusted by the gas flow pressure: higher pressure yields smaller bead and hollow capsule sizes. If the target diameter of the hollow capsules exceeds 400 µm, the size distribution of the hollow capsules can be made more uniform.

### <Controlling the Cell Density within the Hollow Capsules>

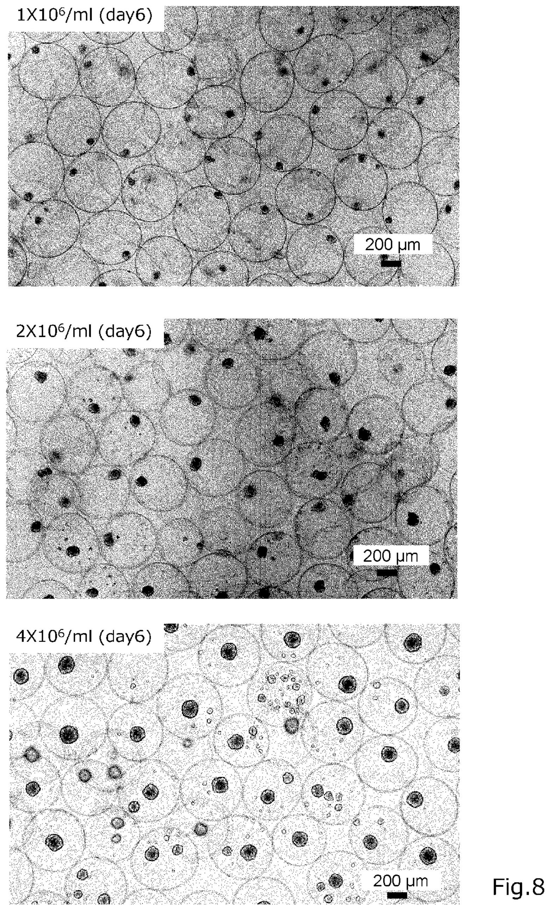

Figure 8 shows microscopic images of organoids prepared using cells at various densities (cells/mL). The black bar at the bottom right of each image indicates a 200 µm scale. Hollow capsules prepared from beads made with a 55 kPa gas flow were used. The diameter of the hollow capsules was greater than 400 µm. Organoid diameter is highly correlated with cell density. When cell density ranges from 1x10⁶ cells/mL to 2x10⁶ cells/mL, a single organoid often forms within a single hollow capsule. In addition, the size distribution of these organoids tends to be more uniform.

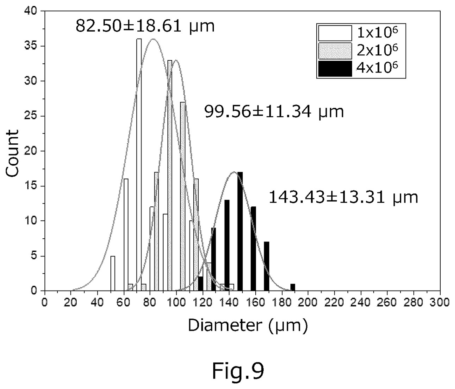

Table 1 and the histogram in Figure 9 show the distribution of organoid diameters. The expression attached to the histogram denotes (mean organoid diameter) ± (standard deviation of organoid diameter) (µm). In the histogram, the horizontal axis represents organoid diameter and the vertical axis represents organoid count. Table 1 shows, for each cell density, the percentage (%) of organoids in each diameter class relative to the total organoid count.

**[Table 1]**

| 55kPa | Diameter (µm) | | | | | | | | |
|---|---|---|---|---|---|---|---|---|---|
| 1X10⁶/ml | 50-60 | 60-70 | 70-80 | 80-90 | 90-100 | 100-110 | 110-120 | 120-130 | 130-140 |
| Percentage (%) | 5 | 17 | 38 | 13 | 12 | 0 | 11 | 0 | 0 |

| 2X10⁶/ml | 60-70 | 70-80 | 80-90 | 90-100 | 100-110 | 110-120 | 120-130 | 130-140 | |
|---|---|---|---|---|---|---|---|---|---|
| Percentage (%) | 1 | 1 | 17 | 33 | 27 | 16 | 4 | 1 | |

| 4X10⁶/ml | 110-120 | 120-130 | 130-140 | 140-150 | 150-160 | 160-170 | 170-180 | 180-190 | |
|---|---|---|---|---|---|---|---|---|---|
| Percentage (%) | 3 | 15 | 21 | 28 | 20 | 11 | 0 | 2 | |

### <Relationship between Hollow Capsule Size and Organoid Size>

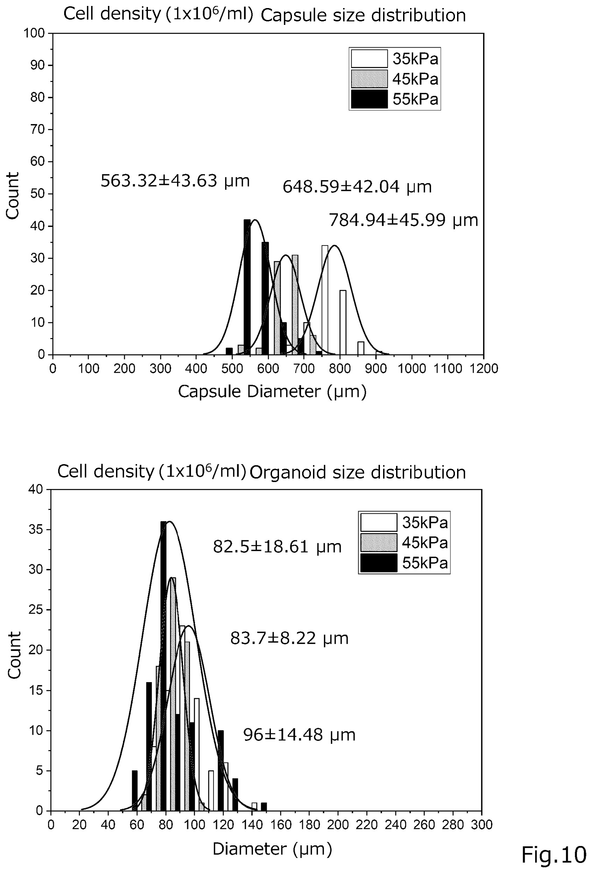

The upper panel of Figure 10 is a histogram of hollow capsule diameters (Capsule Diameter) for organoids produced from cells at a density of 1x10⁶ cells/mL within the beads. Hollow capsules prepared from beads fabricated with gas flows of 35 to 55 kPa were used. Table 2 shows the percentage (%) of hollow capsules in each diameter class relative to the total number of hollow capsules.

**[Table 2]**

| 1x10⁶/ml | Diameter (µm) | | | | | |
|---|---|---|---|---|---|---|
| 35kPa | 650-700 | 700-750 | 750-800 | 800-850 | 850-900 | 900-950 |
| Percentage (%) | 4 | 14 | 47 | 28 | 6 | 1 |

| 45kPa | 500-550 | 550-600 | 600-650 | 650-700 | 700-750 | |
|---|---|---|---|---|---|---|
| Percentage (%) | 4 | 3 | 41 | 44 | 8 | |

| 55kPa | 450-500 | 500-550 | 550-600 | 600-650 | 650-700 | 700-750 |
|---|---|---|---|---|---|---|
| Percentage (%) | 2 | 44 | 37 | 11 | 5 | 1 |

The lower panel of Figure 10 is a histogram of organoid diameters (Diameter) for organoids produced using these hollow capsules with cells at a density of 1x10⁶ cells/mL. Table 3 shows the percentage (%) of organoids in each diameter class relative to the total number of organoids.

**[Table 3]**

| 1X10⁶/ml | Diameter (µm) | | | | | | | | |
|---|---|---|---|---|---|---|---|---|---|
| 35kPa | 70-80 | 80-90 | 90-100 | 100-110 | 110-120 | 120-130 | 130-140 | 140-150 | |
| Percentage (%) | 11 | 21 | 32 | 19 | 7 | 8 | 0 | 1 | |

| 45kPa | 60-70 | 70-80 | 80-90 | 90-100 | 100-110 | 110-120 | 120-130 | 130-140 | |
|---|---|---|---|---|---|---|---|---|---|
| Percentage (%) | 3 | 25 | 40 | 29 | 1 | - | - | - | |

| 55kPa | 50-60 | 60-70 | 70-80 | 80-90 | 90-100 | 100-110 | 110-120 | 120-130 | 140-150 |
|---|---|---|---|---|---|---|---|---|---|
| Percentage (%) | 5 | 17 | 38 | 13 | 12 | 0 | 11 | 4 | 1 |

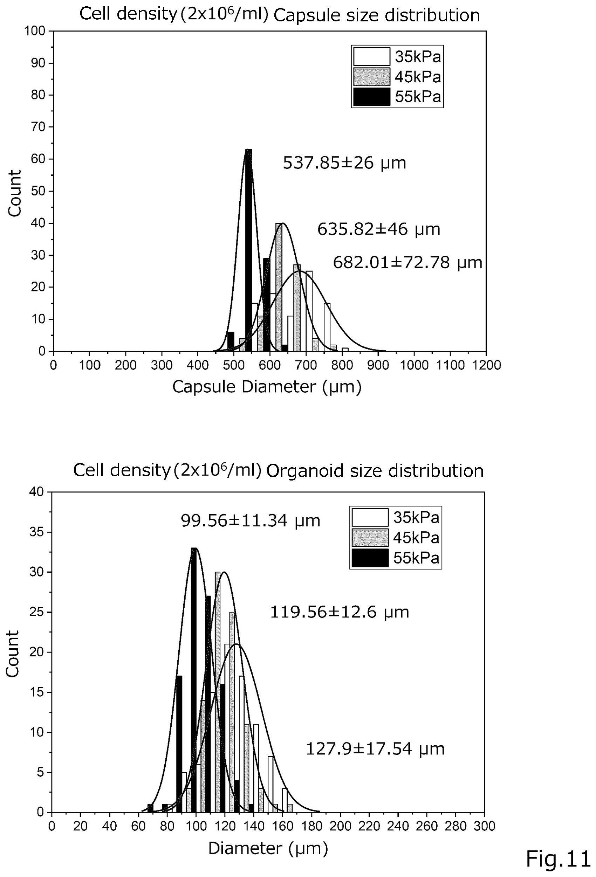

The upper panel of Figure 11 is a histogram of hollow capsule diameters (Capsule Diameter) for organoids produced from cells at a density of 2x10⁶ cells/mL within the beads. Hollow capsules prepared from beads fabricated with gas flows of 35 to 55 kPa were used. Table 4 shows the percentage (%) of hollow capsules in each diameter class relative to the total number of hollow capsules.

**[Table 4]**

| 2x10⁶/ml | Diameter (µm) | | | | | | |
|---|---|---|---|---|---|---|---|
| 35kPa | 500-550 | 550-600 | 600-650 | 650-700 | 700-750 | 750-800 | 800-850 |
| Percentage (%) | 1 | 17 | 20 | 13 | 28 | 17 | 1 |

| 45kPa | 500-550 | 550-600 | 600-650 | 650-700 | 700-750 | 750-800 | |
|---|---|---|---|---|---|---|---|
| Percentage (%) | 5 | 13 | 45 | 31 | 5 | 2 | |

| 55kPa | 450-500 | 500-550 | 550-600 | 600-650 | | | |
|---|---|---|---|---|---|---|---|
| Percentage (%) | 6 | 63 | 29 | 2 | | | |

The lower panel of Figure 11 is a histogram of organoid diameters (Diameter) for organoids produced using these hollow capsules with cells at a density of 2x10⁶ cells/mL. Table 5 shows the percentage (%) of organoids in each diameter class relative to the total number of organoids.

**[Table 5]**

| 2x10⁶/ml | Diameter (µm) | | | | | | | | |
|---|---|---|---|---|---|---|---|---|---|
| 35kPa | 60-70 | 70-80 | 80-90 | 90-100 | 100-110 | 110-120 | 120-130 | 130-140 | |
| Percentage (%) | 1 | 1 | 20 | 38 | 31 | 19 | 5 | 1 | |

| 45kPa | 90-100 | 100-110 | 110-120 | 120-130 | 130-140 | 140-150 | 150-160 | 160-170 | 170-180 |
|---|---|---|---|---|---|---|---|---|---|
| Percentage (%) | 3 | 16 | 34 | 28 | 13 | 3 | 1 | 1 | - |

| 55kPa | 60-70 | 70-80 | 80-90 | 90-100 | 100-110 | 110-120 | 120-130 | 130-140 | |
|---|---|---|---|---|---|---|---|---|---|
| Percentage (%) | 1 | 1 | 17 | 33 | 27 | 16 | 4 | 1 | |

### <Release of Organoids from Hollow Capsules>

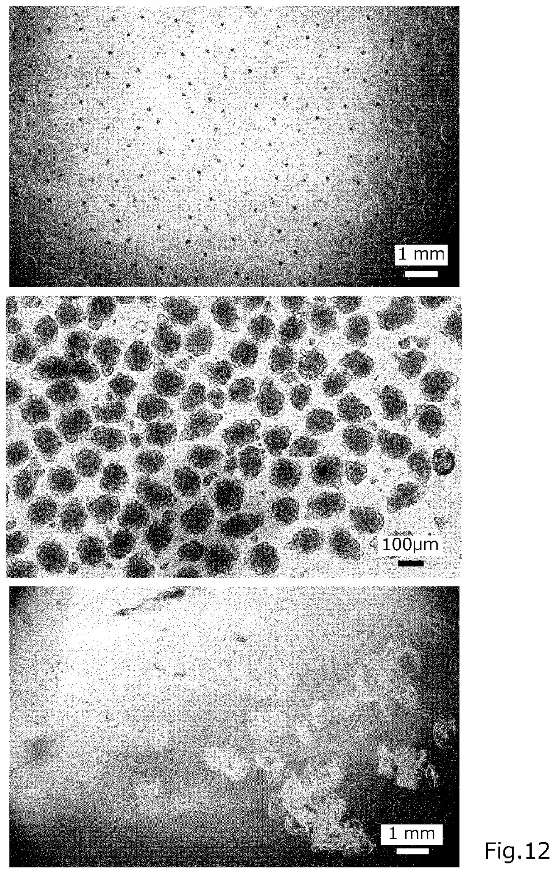

The upper panel of Figure 12 shows a microscopic image of an organoid before release from the hollow capsule. The organoid was obtained by culturing cells inside the hollow capsule for 7 days. First, the hollow capsule was drawn into a syringe through an injection needle whose inner diameter was larger than the capsule's outer diameter (for example, an 18G needle; inner diameter 0.838 ± 0.038 mm). The needle was then replaced with one having an inner diameter smaller than the capsule's outer diameter. The capsule was expelled from the syringe through this smaller-diameter needle, causing the poly-L-lysine membrane that forms the hollow capsule to rupture and release the organoid. A 27G needle was used; its inner diameter was 0.210 ± 0.019 mm. A 23G needle (inner diameter 0.337 ± 0.019 mm) can also be suitably used. The released organoids were then passed through a 200 µm mesh. This leaves the ruptured semipermeable membrane on the mesh while yielding a filtrate containing the organoids. The middle row shows microscopic images of organoids contained in the filtrate. The bottom row shows microscopic images of the hollow capsules remaining on the mesh after organoid release.

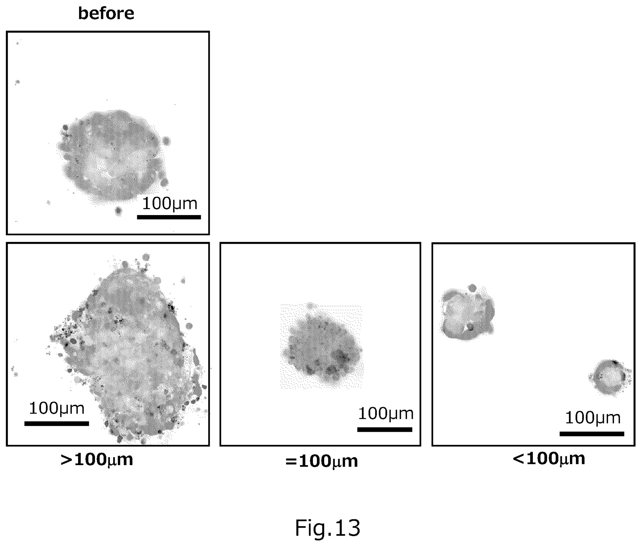

Figure 13 shows fluorescence microscopy images of organoids released from the hollow capsules. The top row shows organoids before release. The three images in the bottom row show organoids after release. For organoids with diameters greater than 100 µm, physical damage to surface cells was observed after processing with the needle and mesh. Therefore, it is preferable that the needle inner diameter and the mesh size be sufficiently large relative to the target organoid outer diameter.

### <Assistance in Aggregation by Mesenchymal Stem Cells>

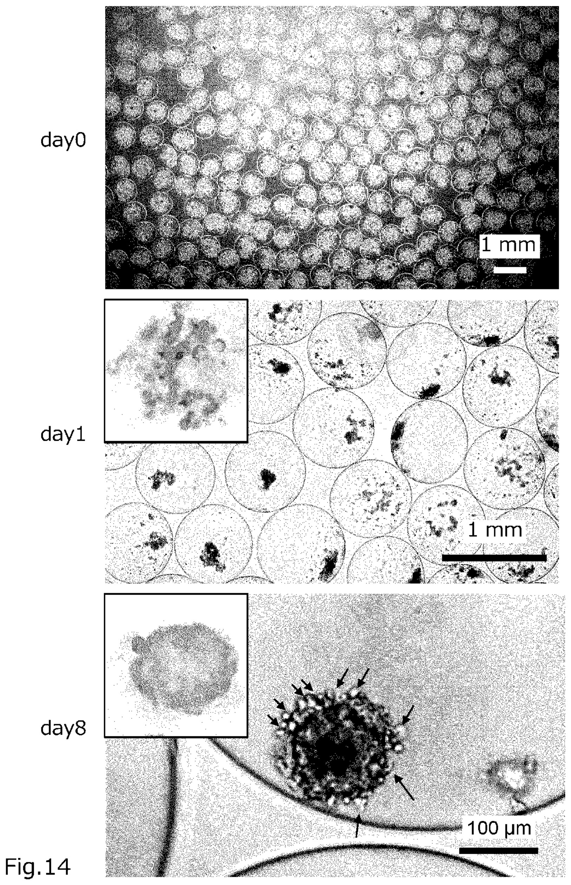

Figure 14 is a microscopic image showing aggregation of cardiomyocytes alone. Only cardiomyocytes were placed in the hollow capsule and incubated for 8 days. The cell density was 3 x 10⁶ cells/mL. The upper-left photographs in the Day 1 and Day 8 images are fluorescence images. On Day 1, the cells had not organized; by Day 8, they had formed organized structures. However, dead cells were frequently observed within the spheroids. The cell viability was 60%. Viability was assessed by observing live- and dead-cell markers under a fluorescence microscope using the LIVE/DEAD^{™} Cell Viability Assay Kit (Thermo Fisher) to stain live and dead cells/tissues. In the figure, live cells are shown in green and dead cells in red. The same convention applies below.

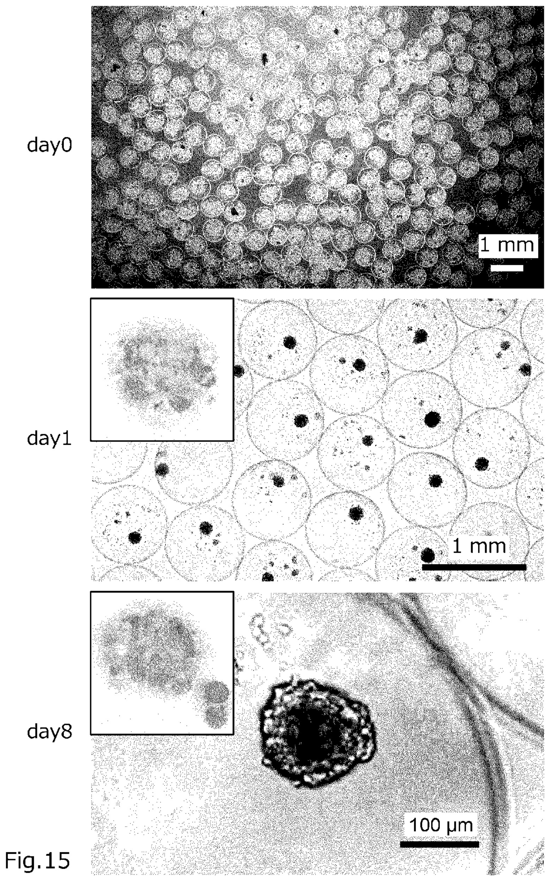

Figure 15 is a microscopic image showing aggregation of a mixture of cardiomyocytes and mesenchymal stem cells. Cardiomyocytes and mesenchymal stem cells were placed in a hollow capsule at a 10:1 ratio and incubated for 8 days. The cell density was 3 x 10⁶ cells/mL. The upper-left photographs for Day 1 and Day 8 are fluorescence images. On Day 1, the cells had already organized; by Day 8, they were well organized. Compared with the cardiomyocyte spheroids shown in Figure 14, the organoids in this figure exhibited a lower frequency of dead cells.

### <Evaluation of Cardiac Myocyte Organoids>

Table 6 summarizes the cell viability (%) from eight trials of the experiments shown in Figures 14 and 15. Before culturing in the hollow capsules, cardiomyocytes (CM) were prepared under the same culture conditions for each trial; however, their Day 0 viability varied between trials. In contrast, Day 0 viability for mesenchymal stem cells (MSCs) was consistently high. The reduced viability observed even at Day 0 is likely due to damage from processing applied to the cells prior to aggregation for example enzymatic or mechanical treatments used to detach or process cells pre-cultured on dishes. Cardiomyocytes appeared to be more sensitive to such damage than mesenchymal stem cells.

As incubation progressed, the viability of cells within the myocardial organoids after 6 days (Day 6) was high. Even when the Day 0 viability of cardiomyocytes was low, high viability was observed after 6 days; this trend was seen, for example, in the fifth and sixth trials. This is thought to reflect that MSCs not only assist CM aggregation but also enhance cell survival within the aggregates.

**[Table 6]**

| Number of trials | Day0 (%) | | Day6 (%) |
|---|---|---|---|
| | iPS-derived myocardial cells | iPS-derived MSC cells | Myocardial organoids |
| Trial 1 | 80.9 | 97.4 | 81.3 |
| Trial 2 | 80.6 | 98.4 | 90.6 |
| Trial 3 | 68.7 | 95.8 | 78.4 |
| Trial 4 | 72.6 | 96.4 | 79.4 |
| Trial 5 | 62.4 | 95.0 | 77.2 |
| Trial 6 | 58.7 | 81.4 | 72.3 |
| Trial 7 | 75.1 | 98.1 | 78.7 |
| Trial 8 | 85.1 | 98.1 | 83.1 |

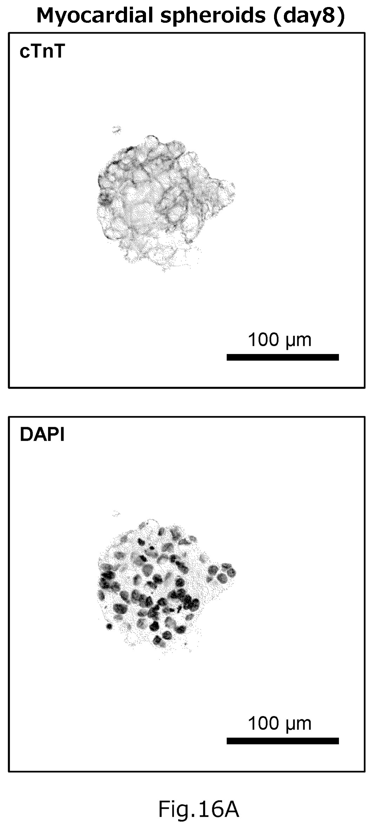

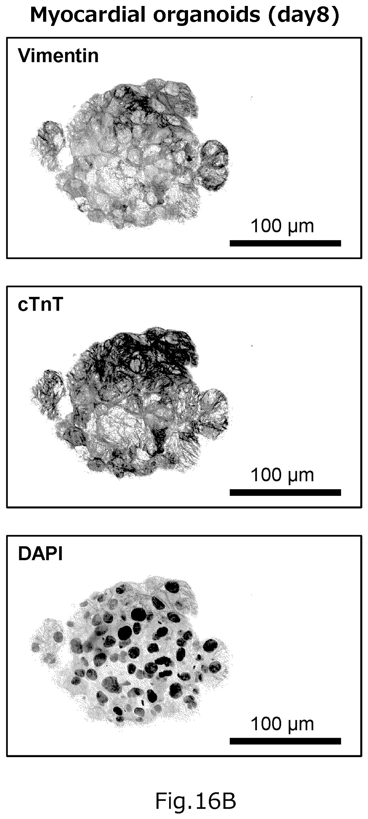

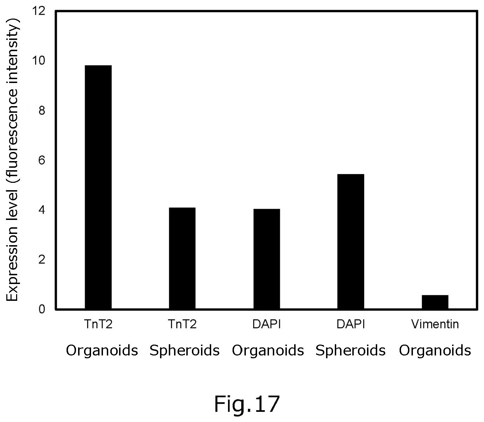

Figure 16A shows microscopic images of myocardial markers in myocardial spheroids. Figure 16B shows microscopic images of myocardial markers in myocardial organoids. The myocardial marker cTnT (TnT2 gene) and the MSC marker vimentin were fluorescently stained. Cell nuclei were additionally stained with DAPI. Figure 17 shows the total fluorescence intensity for each marker in Figures 16A and 16B. Compared with the myocardial spheroids, the myocardial organoids exhibited stronger expression of myocardial markers. Moreover, cardiomyocyte-specific structures were clearly observed in the myocardial organoids. These results indicate that the tissue quality of the myocardial organoids was superior to that of the myocardial spheroids.

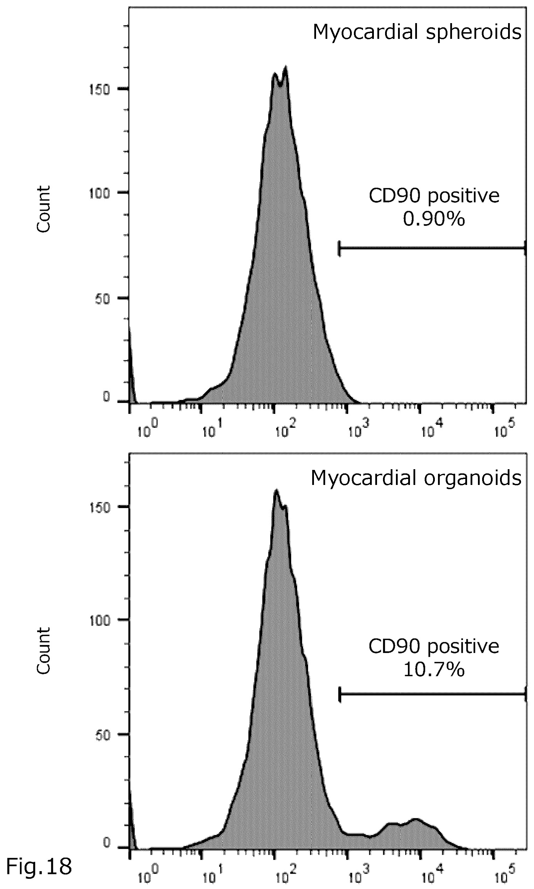

Figure 18 is a histogram of expression intensity for CD90, an MSC marker. Expression intensity was measured by FACS. CD90-positive cells comprised 0.9% of cells in the myocardial spheroids, whereas CD90-positive cells comprised 10.7% of cells in the myocardial organoids. The CM:MSC ratio was set to 10:1 prior to organoid formation; after 6 days of culture, the CM:MSC ratio had changed to 9:1.

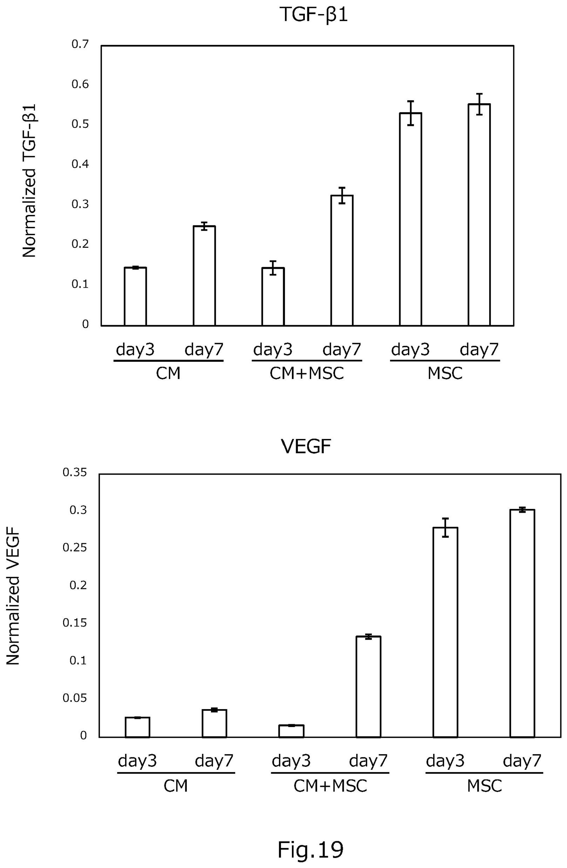

Figure 19 shows graphs of TGF-β1 and VEGF secretion levels, comparing myocardial spheroids (CM), myocardial organoids (CM+MSC), and MSC spheroids. These secreted factors are markers of myocardial tissue. The data were normalized to cell number. Cells within the myocardial organoids secreted higher levels of these factors than cells within the myocardial spheroids, notably on Day 7. In addition, secretion tended to increase with longer culture duration.

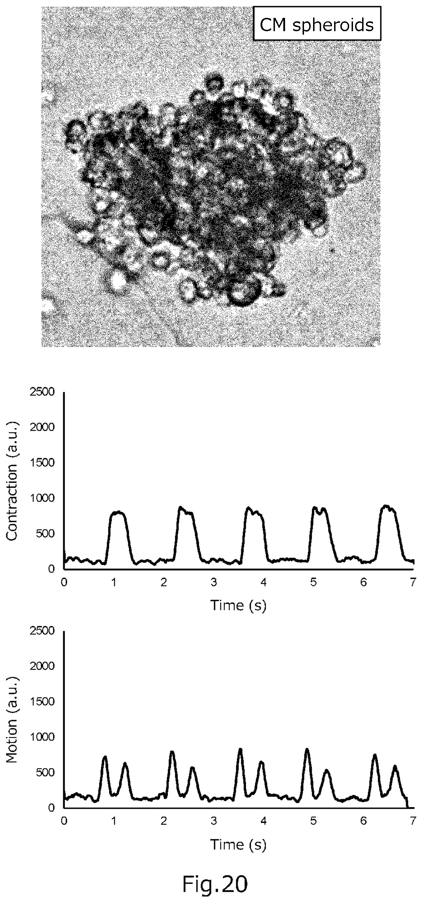

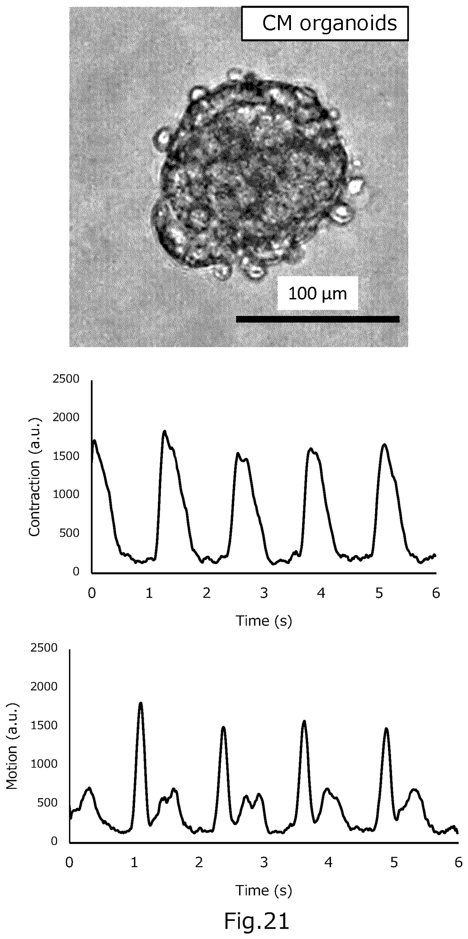

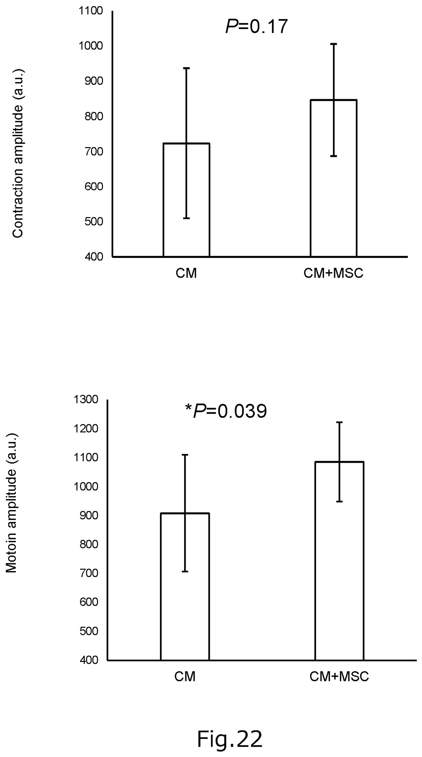

Figure 20 shows the results of contraction force analysis (Contraction & Motion analysis) for myocardial spheroids (CM). Figure 21 shows the results for myocardial organoids (CM). a.u. denotes arbitrary units. The middle panels in each figure show temporal changes in contraction force (Contraction). The lower panels show temporal changes in contraction speed (Motion). The upper panel of Figure 22 shows the mean contraction amplitude, and the lower panel shows the mean motion amplitude. The contraction force and contraction speed of the myocardial organoids were higher than those of the myocardial spheroids.

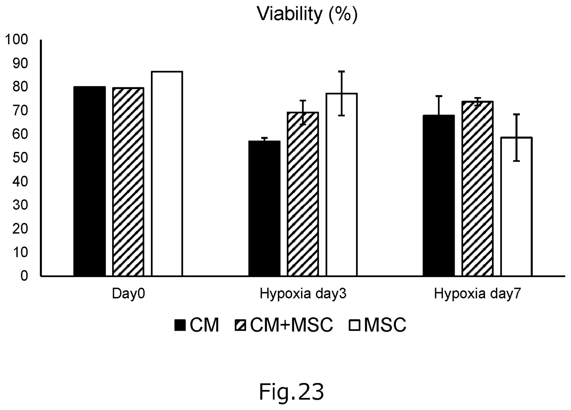

Figure 23 shows analysis results of cell survival under hypoxic conditions. Under hypoxia at 5% O₂, the survival rate of myocardial organoids tended to be higher than that of myocardial spheroids.

### <Comparison with Microwell Plate Culture>

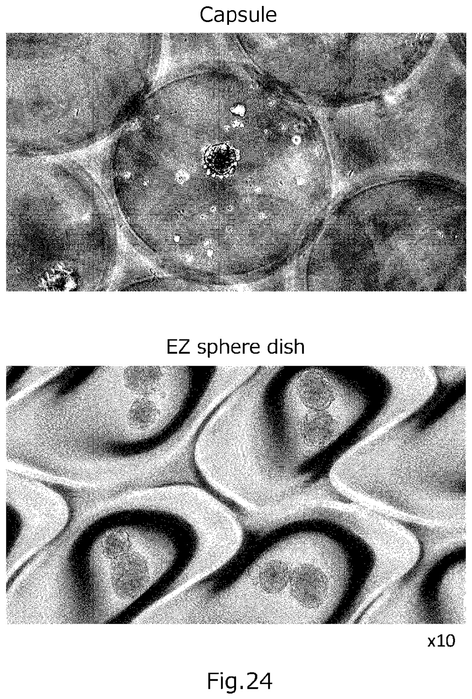

The upper panel of Figure 24 shows microscopic images of myocardial organoids produced using hollow capsules. The lower panel shows microscopic images of myocardial organoids produced using a microwell plate. An EZ sphere dish (trademark) was used as the microwell plate. Static (non-agitated) culture was performed in the microwell plate.

The images in Figure 24 show organoids on Day 2 of culture. After two days, beating cardiomyocyte organoids had formed in both hollow capsules and microwell plates. No significant difference in beating rate was observed between the spheroids produced by the two methods. However, unexpected fusion of cardiomyocyte organoids occurred in the microwell plates. While only one myocardial organoid occupied a single hollow capsule, multiple organoids had entered into each well of the microwell plate; these organoids were expected to fuse over time.

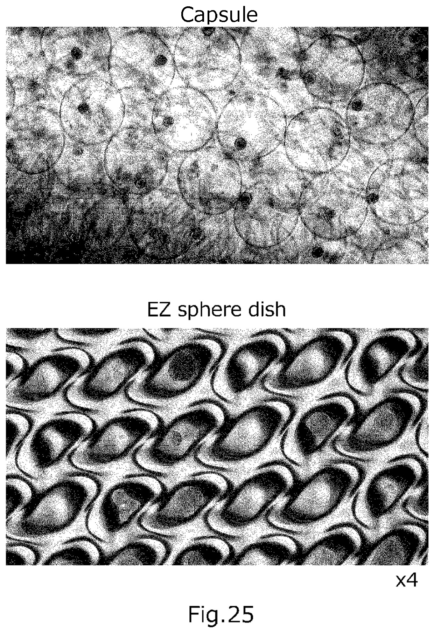

The upper panel of Figure 25 shows microscopic images of myocardial organoids produced in hollow capsules. The lower panel shows images of organoids produced in a microwell plate. These images depict organoids on Day 8 of culture. After 8 days, organoids cultured in the microwell plate had migrated between wells and fused with other organoids. The cause of organoid migration is thought to be vibration of the container or cell migration. As shown in the lower images, organoid sizes were heterogeneous, and some wells lacked organoids. In contrast, organoids generated in the hollow capsules (upper images) were more uniform in size.

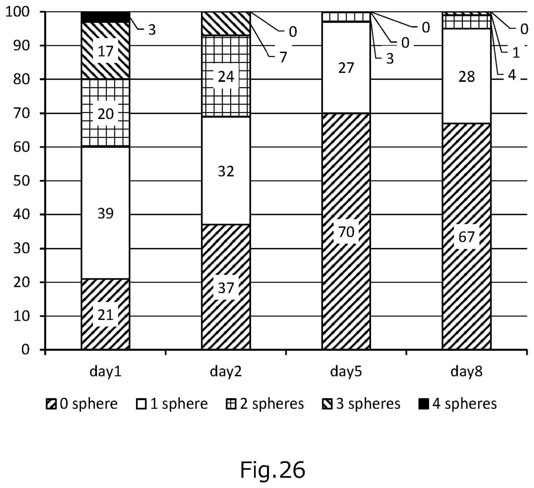

Figure 26 shows the results of counting the number of organoids per well in the microwell plate over time. Wells containing two or more organoids appeared from the early stages of culture. Moreover, as culture time increased, the number of wells without organoids increased.

Table 7 shows the percentage of wells (relative to the total number of wells) containing organoids in each diameter class in the microwell plate. Wells without organoids were included in the count. Organoid diameters were broadly distributed from 40 µm to 300 µm.

**[Table 7]**

| Absence or Presence | Diameter (µm) | | Existence ratio (%) |
|---|---|---|---|
| | Lower limit | Upper limit | |
| Absence | - | | 62 |
| | 40 | 60 | 3 |
| | 61 | 80 | 0 |
| | 81 | 100 | 3 |
| | 101 | 120 | 1 |
| | 121 | 140 | 4 |
| | 141 | 160 | 7 |
| | 161 | 180 | 3 |
| Presence | 181 | 200 | 6 |
| | 201 | 220 | 2 |
| | 221 | 240 | 3 |
| | 241 | 260 | 5 |
| | 261 | 280 | 1 |
| | 281 | 300 | 1 |

From the above, cardiomyocyte organoids were found to fuse readily with one another in microwell plates, leading to heterogeneous sizes. In contrast, as shown in Figures 3-5 and Tables 3 and 5, cardiomyocyte organoids generated in hollow capsules exhibited more uniform sizes.

Table 8 summarizes the cell survival rates (%) for organoids produced using hollow capsules (Capsule) and for organoids produced using a microwell plate (EZ sphere dish). The Day 0 survival rates for cardiomyocyte (CM) spheres and mesenchymal stem cell (MSC) spheres are also summarized. At Days 9 and 16 of incubation, organoids produced in hollow capsules showed survival rates comparable to those of organoids produced in microwell plates (EZ sphere dishes).

**[Table 8]**

| | Survival rates | | |
|---|---|---|---|
| | Day0 | Day9 | Day16 |
| CM sphere/Capsule | 62.6 | - | - |
| MSC sphere/Capsule | 89.7 | - | - |
| Organoid/Capsule | - | 67 | 74.6 |
| Organoid/EZ sphere dish | - | 71.7 | 71.4 |

### <Application to Other Stromal Cells>

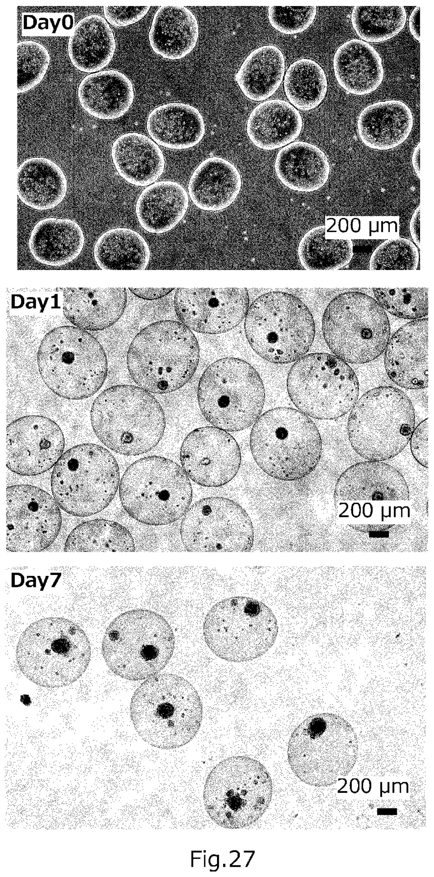

Figure 27 shows a microscopic image of spheres formed by adipose tissue-derived mesenchymal stem cells (AD-MSCs). AD-MSCs, a type of stromal cell, were aggregated using hollow capsules, yielding AD-MSC spheres. Cells within these spheres strongly expressed vimentin, an MSC marker.

### <Application to Other Cells of Interest>

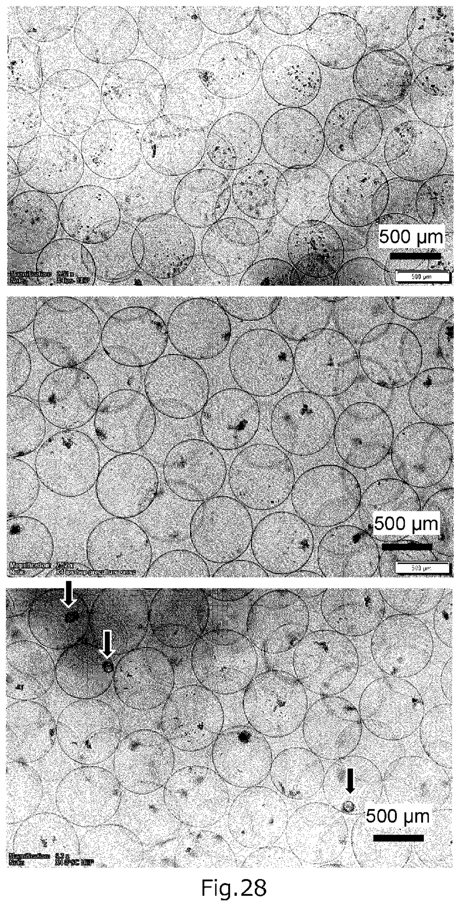

Figure 28 shows a microscopic image of an organoid composed of hepatocyte-like cells derived from iPS cells and mesenchymal stem cells (MSCs). Hepatocyte-like cells and MSCs were aggregated using hollow capsules, producing a liver organoid. Cells within the liver organoid strongly expressed α-1-antitrypsin, a hepatic marker.

### <Discussion>

Encapsulating single or multiple cell types within hollow capsules enables efficient formation of aggregates from these cells. Because the hollow capsules isolate aggregates from one another, this method has advantages over microwell plate-based methods, such as reduced aggregate fusion, compatibility with rotary culture, and fewer constraints on handling and transport of the aggregates.

The size of hollow capsules can be adjusted by controlling the capsule-formation process; preferably, the capsule diameter is greater than 400 µm. The size of the aggregates can be tuned by adjusting the cell density inside the capsules; preferably, aggregate diameters are greater than 70 µm. By appropriately matching the hollow capsule diameter to the target aggregate diameter, highly uniform aggregates can be produced.

Using a continuous bead-production process enables rapid, large-scale manufacture of hollow capsules, facilitating scale-up of aggregate production to an industrial level. For example, throughput can be further increased by employing multiple nozzles for bead production.

Even when cells of interest (e.g., cardiomyocytes) have low viability prior to culture in hollow capsules, satisfactory aggregates can still be obtained. This is an additional benefit of combining cells of interest with auxiliary cells such as MSCs. Using auxiliary cells enables rapid generation of highly viable, highly functional, and well-organized aggregates.

Hollow capsules can be used to generate aggregates from various cell types, including cardiomyocytes, AD-MSCs, and iPS-derived hepatocyte-like cells.

This application is based on Japanese Patent Application No. 2023-204841, filed December 4, 2023, entitled "Method for Producing Cell Aggregates, Population of Aggregates, and Cell Culture Method," and claims the benefit of priority from that Japanese application. The entire contents of the cited Japanese application are incorporated herein by reference.

Explanation of Reference Signs 11: hollow capsule; 12: cell; 13: gel; 14: cells of interest; 15: auxiliary cell; 16: aggregate; 17: cell; 18: nutrient factor; 19: stromal cell; 20: parenchymal cell; 21: aggregate; 22: nozzle; 23: gas flow; 24: beads; 25: liquid; 27: semipermeable membrane; 28: space; 30: tube; 31: population

## Claims

1. A method for producing a cell aggregate, comprising:
suspending cells within a hollow capsule composed of a semipermeable membrane; and
incubating said hollow capsule in a liquid culture medium to aggregate said cells within said hollow capsule, wherein
said cells comprise a mixture of dispersed cells of interest and auxiliary cells that assist in aggregation of the cells of interest, and
the cells of interest aggregate more readily when the auxiliary cells mediate than when they are absent.

2. The method for producing a cell aggregate according to Claim 1, wherein
the cells of interest are cardiomyocytes, hepatocytes, or other parenchymal cells, and/or wherein
the auxiliary cells are anyone of mesenchymal stem cells, fibroblasts, vascular endothelial cells, and other stromal cells.

3. A method for producing a cell aggregate, comprising:
suspending dispersed cells within a hollow capsule composed of a semipermeable membrane; and
incubating said hollow capsule in liquid culture medium to aggregate said cells within said hollow capsule, wherein said cells comprise at least stromal cells.

4. The method for producing a cell aggregate according to Claim 3, wherein
the stromal cells are one or more of mesenchymal stem cells, fibroblasts, vascular endothelial cells, or other stromal cells.

5. The method for producing a cell aggregate according to Claim 4, wherein
the cells further comprise cardiomyocytes, hepatocytes, or other parenchymal cells.

6. The method for producing a cell aggregate according to any one of Claims 1 to 5, further comprising:
encapsulating a gel that contains said cells with said semipermeable membrane, wherein the gel is made of a material different from that of the semipermeable membrane;
dissolving the gel; and
allowing the dissolved gel to diffuse outward through the semipermeable membrane to produce the hollow capsule, wherein said cells are retained within the hollow capsule.

7. The method for producing a cell aggregate according to Claim 6, wherein
the gel comprises alginate cross-linked with divalent cations, and
the gel is dissolved by applying a chelating agent.

8. The method for producing a cell aggregate according to Claim 6, further comprising
forming beads of the gel containing the cells by extruding the gel while sheathing it with a gas stream, and subsequently encapsulating the beaded gel with the semipermeable membrane.

9. The method for producing a cell aggregate according to any one of Claims 1 to 5, wherein
the hollow capsule is ruptured by passing it through a tube having an inner diameter smaller than the outer diameter of the hollow capsule, and
the aggregated cells are separated and recovered from the ruptured hollow capsule.

10. The method for producing a cell aggregate according to Claim 9, wherein
the semipermeable membrane comprises poly-L-lysine.

11. The method for producing a cell aggregate according to any one of Claims 1 to 5, further comprising
incubating the hollow capsules with rotary stirring.

12. The method for producing a cell aggregate according to Claim 1, further comprising
proliferating the aggregated cells by continued incubation of the hollow capsules.

13. A population of aggregates, wherein:
the mean diameter of aggregates in the population is 70 to 150 µm, and
the standard deviation of the diameters of the aggregates in the population is 7 to 20 µm; wherein the aggregates are either
(a) aggregates formed by cells of interest aggregating with auxiliary cells interposed between them, the cells of interest aggregating more readily when auxiliary cells mediate than when they are absent, or
(b) aggregates formed by aggregation of stromal cells.

14. The population of aggregates according to Claim 13, wherein
each aggregate is individually enclosed within a hollow capsule formed from a semipermeable membrane, and
each aggregate is suspended in the liquid contained within its hollow capsule.

15. A method for culturing cells, comprising:
suspending cells within hollow capsules composed of a semipermeable membrane; and
incubating the hollow capsules in liquid culture medium until the cells aggregate within the hollow capsules, wherein
the cells comprise a mixture of dispersed cells of interest and auxiliary cells that assist aggregation of the cells of interest, and
the cells of interest aggregate more readily when the auxiliary cells mediate than when they are absent.

16. A method for culturing cells, comprising:
suspending dispersed cells within a hollow capsule formed of a semipermeable membrane; and
incubating the hollow capsule in liquid culture medium until the cells aggregate within the hollow capsule, wherein
the cells comprise at least stromal cells.
